# EUROPÄISCHE PATENTSCHRIFT

(11) **EP 2 310 864 B1**
(45) Veröffentlichungstag und Bekanntmachung des Hinweises auf die Patenterteilung: **29.01.2014**
(21) Anmeldenummer: 09799916.3
(22) Anmeldetag: 23.07.2009
(51) Int. Cl.: G01N 33/86

(54) **VERFAHREN ZUR PRÄOPERATIVEN BESTIMMUNG DES INTRAOPERATIVEN BLUTUNGSRISIKOS EINES PATIENTEN**
METHOD FOR THE PREOPERATIVE DETERMINATION OF THE INTRAOPERATIVE RISK OF BLEEDING OF A PATIENT
PROCÉDÉ DE DÉTERMINATION PRÉ-OPÉRATOIRE DU RISQUE DE SAIGNEMENT INTRA-OPÉRATOIRE D 'UN PATIENT

(30) Priorität: 24.07.2008 CH 11572008
(43) Veröffentlichungstag der Anmeldung: 20.04.2011
(73) Patentinhaber: Zentrum für Labormedizin, 9001 St. Gallen (CH)
(72) Erfinder: KORTE, Wolfgang, 9007 St. Gallen (CH)
(74) Vertreter: Hasler, Erich
(86) Internationale Anmeldenummer: PCT/CH2009/000262
(87) Internationale Veröffentlichungsnummer: WO 2010/009568

(56) Entgegenhaltungen:
- DE-B4- 19 833 844
- KORTE WOLFGANG ET AL: "Preoperative fibrin monomer measurement allows risk stratification for high intraoperative blood loss in elective surgery." THROMBOSIS AND HAEMOSTASIS JUL 2005, Bd. 94, Nr. 1, Juli 2005 (2005-07), Seiten 211-215, XP008103192 ISSN: 0340-6245
- TERUYA ET AL: "A Normal aPTT Does Not Guarantee Adequate Coagulation Factor Levels" ANESTHESIOLOGY, [Online] Bd. 94, Nr. 3, März 2001 (2001-03), Seite 542, XP002518609 Gefunden im Internet: URL:http://journals.lww.com/anesthesiology /pages/articleviewer.aspx?year=2001&issue= 03000&article=00035&type=fulltext> [gefunden am 2009-03-05]

## Beschreibung

Die Erfindung betrifft ein Verfahren zur präoperativen Bestimmung des Risikos eines Patienten zur intraoperativen Blutungsneigung wie beansprucht.

Bei chirurgischen Eingriffen können sich Komplikationen durch intraoperative Blutungen ergeben. Wenn diffuse Blutungen im Wundbereich oder an den Wundrändern auftreten, ohne dass eine ersichtliche mechanische Ursache vorliegt, so ist von einer Blutungsneigung aufgrund einer Koagulopathie auszugehen.

Zur Abklärung von Blutungen oder Gerinnungsproblemen sind bereits verschiedene Tests bekannt. Ein häufig verwendeter Test ist als PTT- oder aPTT-Test (activated partial thromboplastin time) bekannt. Dieser misst die Zeitspanne (in Sekunden) die zur Bildung eines Gerinnsels im Reagenzglas benötigt wird. Zu diesem Zweck wird eine Zitratblutprobe zentrifugiert und das überstehende Plasma verwendet.

Ein anderer Test ist der PT-Test (prothrombin time), der bei Durchführung als Ratio gegenüber einem Normalplasma als Quick-Test bezeichnet wird.

Die aPTT- und PT-Tests sind in der Vergangenheit auch schon zur Bestimmung des Risikos zur intraoperativen Blutungsneigung herangezogen worden. Verschiedene Studien haben aber gezeigt, dass diese Tests dafür nur schlecht geeignet sind. So wurde in einem im Jahre 2005 publizierten Artikel ("Preoperative fibrin monomer measurement allows risk stratification for high intraoperative blood lective surgery" in THROMBOSIS & HAEMOSTASIS, Jul. 2005, Bd. ,1, Seiten 211) festgestellt, dass intraoperativer Blutverlust keine Relation mit der Prothrombin-Zeit (prothrombin time), den aPTT - Werten und der Blutplättchenzahl hat.

In einem von Teruya et al. verfassten Artikel mit dem Titel "A normal aPTT does not guarantee adequate coagulation factor levels" (in ANESTHESIOLOGY, Bd. 94, Nr. 3, März 2001, Seite 542) wird dargelegt, dass eine normale PTT nicht voraussagt, dass auch eine normale Gerinnungsfaktoraktivität besteht. Es wird festgestellt, dass die aPTT nicht als prädiktiver Faktor für einen erniedrigten Gerinnungsfaktorlevel eingesetzt werden kann, sondern im Gegenteil unzuverlässig sei. Damit kann die Verwendung der aPTT präoperativ nicht empfohlen werden. Auch wird im erwähnten Artikel insbesondere kein Zusammenhang zwischen aPTT und der intraoperativen Blutungsneigung hergestellt.

Patienten mit einem erhöhten intraoperativen Blutungsneigung zeigen eine erhöhte Gerinnungsaktivierung. Der Wert der aPTT ist unter anderem auch vom Ausmass der Gerinnungsaktivierung beeinflusst (Ten Boekel E, Bartels P. Abnormally short activated partial thromboplastin times are related to elevated plasma levels of TAT, F1+2, D-dimer and FVIII:C. Pathophysiol Haemost Thromb. 2002;32:137-142).

Es ist bekannt, dass Fibrinmonomer (FM) zur Ermittlung eines "präthrombotischen Zustandes", also zu einer frühen Detektion und dem Monitoring von koagulationsaktivierenden Prozessen dienen kann; dies kommt zum Beispiel vor bei der tiefen Venenthrombose (TVT) oder der disseminierten intravaskulären Gerinnung (DIG bzw. DIC). Fibrinmonomer (FM) eignet sich allerdings auch zum Nachweis eines erhöhten Risikos von intraoperativen Blutungen. So wird in der DE 198 33 844 die Verwendung von Fibrinmonomer als diagnostischer Marker des Risikos eines Patienten zu intraoperativen Blutungen beschrieben. Es wurde festgestellt dass von den untersuchten potentiellen Markern Fibrinmonomere am geeignetsten ist, um präoperativ das Risiko einer intraoperativen Blutungsneigung zu beurteilen.

Entsprechend hat sich die Fibrinmonomer-Bestimmung (FM) als präoperatives Screening zur Beurteilung der intraoperativen Blutungsneigung im Rahmen klinischer Studien bereits bewährt.

Allerdings besteht weiterhin ein Bedürfnis, die Voraussagequalität des Tests zu verbesseren. Es ist daher Aufgabe der vorliegenden Erfindung das beschriebene präoperative Screening weiter zu verbessern.

Wie oben erwähnt ist die aPTT teilweise vom Ausmass der Gerinnungsaktivierung abhängig, isoliert aber nicht zur Risikostratifizieung einer intraoperativen Blutungsneigung verwendbar. Andererseits zeigt die Fibrinmonomerbestimmung bereits ein gutes Potential zur präoperativen Risikostratifizierung einer intraoperativen Blutungsneigung.

Die Aufgabe wird dadurch gelöst dass beide Untersuchungsmethoden kombiniert werden und in einer Probe des Patienten sowohl der Gehalt an Fibrinmonomer (FM) als auch die aktivierte partielle Thromboplastinzeit (aPTT) bestimmt wird. Im Vergleich zum bekannten Verfahren, bei dem lediglich der Gehalt an Fibrinmonomer (FM) geprüft wird, führt das erfindungsgemässe Verfahren zu einer erhöhten diagnostischen Sensitivität, also der Fähigkeit die tatsächlichen Risikopatienten zu erfassen. Gleichzeitig steigt auch die Spezifität; die Verbesserung eines Assays, die sowohl zur Verbesserung der Sensitivität als auch der Spezifität führt, ist ein anzustrebendes aber leider selten erreichtes Ziel. Die hier präsentierte Lösung der Kombination von aPTT und Fibrinmonomer erlaubt das Erreichen dieses Ziels.

Vorteilhaft wird Fibrinmonomer (FM) immunologisch bestimmt. Als Probe für die Bestimmung des Gehalts an Fibrinmonomer als auch zur Bestimmung der aktivierten Thromboplastinzeit (aPTT) wird bevorzugt Citrat-Plasma verwendet.

Die Erfindung betrifft also auch die Verwendung von Fibrinmonomer (FM) in Kombination mit der aktivierten partiellen Thromboplastinzeit (aPTT) als diagnostischer Marker bei der Abschätzung des Risikos einer intraoperativen Blutungsneigung.

### Beispiel 1: Bekannte Fibrin-Monomer-Bestimmung als präoperatives Screening zum Ausschluss einer intraoparativen Blutungsneigung

Bei 226 konsekutiven Patienten mit verschiedenen Eingriffen ohne extrakorporale Zirkulation und mit Arteriensonde wurden prospektiv die präoperative Fibrin-Monomer-Konzentration (FM) untersucht und mit dem Auftreten einer intraoperativen Störung der Hämostase (ISH) korreliert. Das Patientenkollektiv wurde bereits in Korte et al., Clin. Chem. Lab. Med.1998, 36 (4), 235-240 beschrieben.

Die Probenentnahme erfolgte nach Verwerfen der ersten 3 ml aus mit 0.9% NaCl-Lösung gespülten Arteriensonden in 0.125 M Na-Citrat (9+1). Als ISH wurde dabei das Auftreten von diffusen Blutungen im Wundbereich oder an den Wundrändern ohne ersichtliche mechanische Ursache, nachdem bereits eine adäquate lokale Hämostase erzielt worden war, definiert. Fibrinmonomer wurde mit dem Enzymun-Test® FM auf einen ES-300 Gerät bestimmt.

| | FM (µg/ml) | FM (µg/ml) | D-Dimer Tinaquant (mg/l) | | D-Dimer Latex (mg/l) | |
|---|---|---|---|---|---|---|
| | 75te Perzentile | 90te Perzentile | 75te Perz. | 90te Perz. | 75te Perz. | 90te Perz. |
| Perzentile von Patienten ohne ISH | 14.50 | 40.50 | 0.97 | 2.30 | 0.75 | 1.50 |
| RR¹ für ISH | 3.79 | 3.16 | 1.56 | 2.56 | 2.88 | 2.21 |
| OR² | 4.44 | 3.77 | 1.64 | 2.94 | 3.29 | 2.46 |
| Sensitivität | 60% | 30% | 35% | 25% | 45% | 20% |
| Spezifität | 75% | 90% | 75% | 90% | 80% | 91% |
| Pos. prädiktiver Wert | 19% | 22% | 12 % | 19% | 18% | 17% |
| Neg. prädiktiver Wert | 95% | 96% | 92% | 92% | 94% | 92% |

| | | | | | | |
|---|---|---|---|---|---|---|
| ¹ RR: relatives Riskio ² OR: Odds ratio | | | | | | |

| Cut Off FM (µg/ml) | 12 | 11 | 10 | 9 | 8 | 7 | 6 | 5 | 4 |
|---|---|---|---|---|---|---|---|---|---|
| Werte > cut-off bei Patienten ISH vs. non-ISH | 12/20: 63/206 | 12/20: 66/206 | 13/20: 71/206 | 14/20: 78/206 | 14/20: 88/206 | 14/20: 101/206 | 15/20 110/206 | 17/20: 121/206 | 18/20: 137/206 |
| Sensitivität (%) | 60 | 60 | 65 | 70 | 70 | 70 | 75 | 85 | 90 |
| Spezifität (%) | 69 | 68 | 65 | 62 | 57 | 51 | 47 | 41 | 33 |
| Pos. prädikativer Wert (%) | 16 | 15 | 15 | 15 | 14 | 12 | 12 | 12 | 12 |
| Neg. prädikativer Wert (%) | 95 | 95 | 95 | 95 | 95 | 95 | 95 | 97 | 97 |

### Beispiel 2: Kombination von präoperativer aPTT-und Fibrin-Monomer-Bestimmung zum verbesserten Ausschluss einer intraoperativen Blutungsneigung

In einem zweiten Ansatz wurde aus oben beschriebener Population bei 154 evaluierbaren Patienten neben dem FM ausserdem die aPTT mit Pathromtin SL auf einem BCS Gerät bestimmt. FM und aPTT wurden beide im Batch-Verfahren aus bei -80°C gelagerten Aliquots der Proben bestimmt und aus den Messwerten von aPTT und FM eine Ratio gebildet. Ausserdem wurde bei diesen Patienten die 75te Perzentile des intraoperativen Blutverlustes berechnet, die bei 500 ml lag. Die gemeinsame präoperative Verwendung der aPTT in einer Ratio mit den FM Werten erlaubt am nach ROC Kurve optimalen Punkt (10.7252) mit einer Sensitivität von 94.44%, einer Spezifität von 52.68%, einem positiv prädiktiven Wert von 39.1 % und einem negativ prädiktiven Wert von 96.7% einen intraoperativen Blutverlust von mehr als 500 ml, also oberhalb der 75ten Perzentile, vorauszusagen.

Zur Bestimmung des optimalen Punktes wurden Sensitivität, Spezifität, positiver (PPV) und negativer prädiktiver Wert (NPV) bei verschiedenen Werten des Verhältnisses zwischen aPTT und FM ("Kriterium") untersucht und in Tabelle 1 dargestellt. Der besseren Übersicht halber wurden die Punkte dargestellt, die eine Sensitivität von 80% bis 100% ergaben.

Daraus wird ersichtlich dass der Wert von 10.7252 den otimalsten Punkt darstellt wenn Sensitivität, Spezifität, positiver und negativer prädiktiver Wert berücksichtigt werden. Es wird auch ersichtlich, dass durch Verschiebung des Punktes vor allem eine höhere Sensitivität bzw. ein höherer negativ prädiktiver Wert (bis 100%) erreicht werden kann.

**Tabelle 1**

| Kriterium | Sensitivität | Spezifität | PPV | NPV |
|---|---|---|---|---|
| <=9.0515 | 80.56 | 58.04 | 38.2 | 90.3 |
| <=9.3871 | 83.33 | 58.04 | 39.0 | 91.5 |
| <=9.4041 | 83.33 | 57.14 | 38.5 | 91.4 |
| <=9.4921 | 83.33 | 56.25 | 38.0 | 91.3 |
| <=9.5074 | 86.11 | 56.25 | 38.7 | 92.6 |
| <=9.7361 | 86.11 | 55.36 | 38.3 | 92.5 |
| <=9.7561 | 86.11 | 54.46 | 37.8 | 92.4 |
| <=9.7619 | 86.11 | 53.57 | 37.3 | 92.3 |
| <=9.8095 | 88.89 | 53.57 | 38.1 | 93.7 |
| <=10.1905 | 91.67 | 53.57 | 38.8 | 95.2 |
| <=10.7059 | 91.67 | 52.68 | 38.4 | 95.2 |
| <=10.7252 * | 94.44 | 52.68 | 39.1 | 96.7 |
| <=10.8127 | 94.44 | 51.79 | 38.6 | 96.7 |
| <=10.8257 | 94.44 | 50.89 | 38.2 | 96.6 |
| <=10.8609 | 94.44 | 50.00 | 37.8 | 96.6 |
| <=11.0744 | 94.44 | 49.11 | 37.4 | 96.5 |
| <=11.3704 | 97.22 | 49.11 | 38.0 | 98.2 |
| <=11.8321 | 97.22 | 48.21 | 37.6 | 98.2 |
| <=11.9929 | 97.22 | 47.32 | 37.2 | 98.1 |
| <=12.1254 | 97.22 | 46.43 | 36.8 | 98.1 |
| <=12.439 | 97.22 | 45.54 | 36.5 | 98.1 |
| <=12.4701 | 100.00 | 45.54 | 37.1 | 100.0 |

Die entsprechenden Werte für die alleinige Verwendung des FM Wertes ergibt eine Sensitivität von 91.67%, einer Spezifität von 51.75%, einem positiv prädiktiven Wert von 37.5% und einem negativ prädiktiven Wert von 95.2%

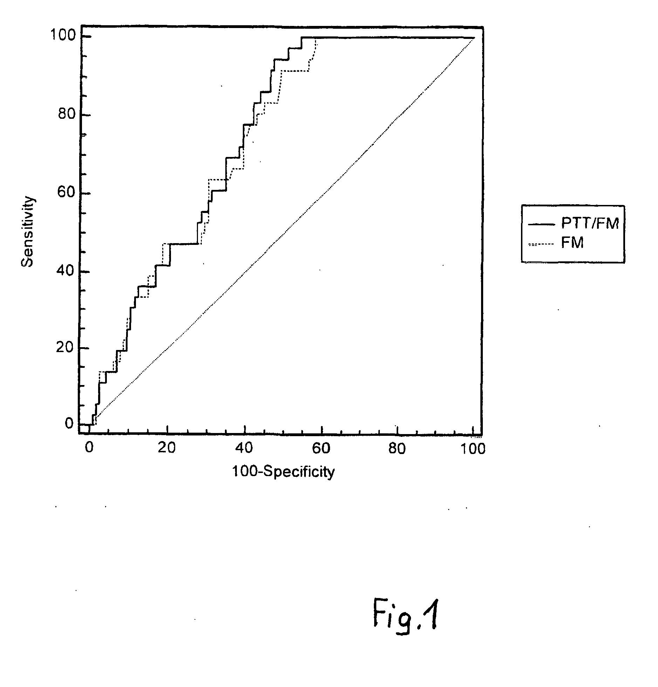

Fig. 1 zeigt eine ROC-Kurve mit den Werten für Fibrinmonomer (FM) allein und den Werten für die Kombination von partieller Thromboplastinzeit (PTT) und Fibrinmonomer (FM).

Receiver operating characteristic (ROC) oder ROC-Kurve Kurven dienen in der Medizin der Bewertung diagnostischer Tests (Ulrich Abel: Bewertung diagnostischer Tests, Hippokrates Verlag, Stuttgart 1993). Die Sensitivität stellt die Fähigkeit einer diagnostischen Methode dar, die tatsächlichen Risikopatienten zu identifizieren. Die Spezifität stellt die Fähigkeit einer diagnostischen Methode dar, falsch positive Tests zu vermeiden. An Fig. 1 ist nun ersichtlich, dass die Kombination eines PTT- und FM-Tests bessere Resultate liefert als ein FM-Test allein, d.h. wenn die Messresultate zueinander in Relation gesetzt werden.

## Patentansprüche

1. Verfahren zur präoperativen Bestimmung des Risikos eines Patienten zur intraoperativen Blutungsneigung,
**dadurch gekennzeichnet,**
**dass** in einer Probe des Patienten sowohl der Gehalt an Fibrinmonomer (FM) als auch die partielle Thromboplastinzeit(PTT) bestimmt und in Relation zueinander gesetzt wird, indem ein Verhältnis aus den Messwerten von partieller Thromboplastinzeit und Fibrinmonomer gebildet wird.

2. Verfahren nach Anspruch 1, **dadurch gekennzeichnet, dass** Fibrinmonomer (FM) immunologisch bestimmt wird.

3. Verfahren nach Anspruch 1 oder 2, **dadurch gekennzeichnet**, es sich bei der Probe um eine Blutprobe handelt.

4. Verfahren nach Anspruch 1 oder 2, **dadurch gekennzeichnet dass** es sich um eine Plasmaprobe handelt.

5. Verwendung von Fibrinmonomer (FM) in Kombination mit der partiellen Thromboplastinzeit (PTT) als diagnostischer Marker bei der Abschätzung des Risikos einer intraoperativen Blutungsneigung, indem ein Verhältnis aus den Messwerten von partieller Thromboplastinzeit und Fibrinmonomer gebildet wird.

## Claims

1. Method for the preoperative determination of the risk of a patient to the intraoperative tendency to bleed,
**characterized in**
**that** the fibrin monomer content (FM) as well as the partial thromboplastin time (PTT) are determined in a sample and are put into relation with one another, a ratio being formed from the measured values of partial thromboplastin time and fibrin monomer.

2. Method according to claim 1, **characterized in that** fibrin monomer is determined immunologically.

3. Method according to claim 1 or 2, **characterized in that** the sample is a blood sample.

4. Method according to claim 1 or 2, **characterized in that** the sample is a plasma sample.

5. Use of fibrin monomer (FM) combined with the partial thromboplastin time (PTT) as a diagnostic marker in the estimation of the risk of an intraoperative tendency to bleed, a ratio being formed from the measured values of partial thromboplastin time and fibrin monomer.

## Revendications

1. Procédé pour la détermination pré-opératoire du risque de saignement intra-opératoire d'un patient,
caractérisé en ce
le temps de thromboplastine partielle (PTT) ainsi que la teneur en monomère de fibrine (FM) sont déterminés dans un échantillon et mis en relation l'un avec l'autre, cependant qu'un rapport est formé à partir des valeurs de mesure du temps de thromboplastine partielle et de monomère de fibrine.

2. Procédé selon la revendication 1, **caractérisé en ce que** le monomère de fibrine (FM) est déterminé de manière immunologique.

3. Procédé selon la revendication 1 ou 2, **caractérisé en ce qu'**il s'agit, pour l'échantillon, d'un échantillon de sang.

4. Procédé selon la revendication 1 ou 2, **caractérisé en ce qu'**il s'agit d'un échantillon de plasma.

5. Utilisation de monomère de fibrine (FM) en combinaison avec le temps de thromboplastine partielle (PTT) comme marqueur de diagnostic pour l'estimation du risque d'une tendance au saignement intra-opératoire, cependant qu'un rapport est formé à partir des valeurs de mesure du temps de thromboplastine partielle et de monomère de fibrine.
